# EUROPÄISCHE PATENTANMELDUNG

(11) **EP 1 576 950 A2**
(43) Veröffentlichungstag der Anmeldung: **21.09.2005**
(21) Anmeldenummer: 05101744.0
(22) Anmeldetag: 07.03.2005
(51) Int. Cl.: A61K 7/48

(54) **Tapioka in kosmetischen Zubereitungen**

(30) Priorität: 19.03.2004 DE 102004013633
(71) Anmelder: Beiersdorf AG, 20253 Hamburg (DE)
(72) Erfinder: Kröpke, Rainer, 22869, Schenefeld (DE); Nielsen, Jens, 24558, Henstedt-Ulzburg (DE); Syskowski, Boris, 22303, Hamburg (DE); Lindemann, Wiebke, 20257, Hamburg (DE); Schäfer, Andreas, 22299, Hamburg (DE); Heinecke, Silke, 21031, Hamburg (DE)

(57) **Zusammenfassung**

Die vorliegende Erfindung betrifft die Verwendung von Tapioka in kosmetischen Zubereitungen.

## Beschreibung

Die vorliegende Erfindung betrifft die Verwendung von Tapioka in kosmetischen Zubereitungen.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Die Hautreinigung erfolgt in der Regel mit Hilfe von oberflächenaktiven Zubereitungen (Seifen, Tenside, seltener alkoholische Zubereitungen), die in Form von schaumbildenen Gelen oder Feststoffen (Seifenstücke) vorliegen und nach dem Auftragen auf die Haut mit Wasser wieder abgespült werden.
Hautpflegeprodukte, in der Regel Cremes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Neben der Reinigung und Pflege der Haut haben Kosmetika auch eine ästhetische Aufgabe. Sie sollen das äußere Erscheinungsbild des Anwenders entsprechend den jeweiligen kulturellen Vorstellungen "verbessern". Kosmetika erfüllen damit eine psychologisch-soziale Funktion, da sie die (optische) Attraktivität der Anwender erhöhen. In diesen Bereich fällt vor allen Dingen die "dekorative" Kosmetik, die mit Hilfe von auf die Haut aufgetragenen Farbstoffen das Erscheinungsbild der Anwender verändert. Indirekt haben aber auch Reinigungs- und Pflegeprodukte einen positiven Einfluss, da eine saubere, gesunde Haut dem Schönheitsideal der Menschen entspricht.

Von besonderer Bedeutung ist in diesem Zusammenhang ist das Erscheinungsbild der Gesichtshaut, da das Gesicht als "Aushängeschild" des Menschen, von der Umwelt in besonderem Maße wahrgenommen wird. Insbesondere auf der Gesichtshaut haben die Reinigungs- und Pflegeprodukte neben der klassischen dekorativen Kosmetik wie Lidschatten und Lippenstift, eine ausgeprägt ästhetische Funktion. So gibt es beispielsweise eine ganze Vielzahl an Zubereitungen zur Behandlung und Prophylaxe von unreiner Haut bzw. Aknehaut.

Aber auch fettige Haut beziehungsweise stark glänzende Hautpartien, sogenannte T-Zonen, werden als optisch unattraktiv empfunden. Zur Behandlung glänzender Hautpartien wurden bisher Zubereitungen angeboten, die eine Reihe von Puderrohstoffen, d.h. partikuläre Rohstoffe wie beispielsweise Distärkephosphat oder Cyclodextrine, enthalten, welche das Sebum absorbieren. Derartige Zubereitungen haben jedoch den Nachteil, dass sie der Haut Feuchtigkeit (Wasser) entziehen und damit zu einem trockenen Hautgefühl führen.

Es war daher die Aufgabe der vorliegenden Erfindung, die Mängel des Standes der Technik zu beseitigen und Zubereitungen zu entwickeln, die einerseits den Hautglanz insbesondere von fettiger Haut wirkungsvoll und lang anhaltend reduzieren ohne der Haut übermäßig große Mengen an Wasser zu entziehen und damit zu einem (subjektiv) trockenen Hautgefühl zu führen.

Überraschend gelöst wird die Aufgabe durch
- die Verwendung von Tapioka in kosmetischen Zubereitungen zur Reduzierung des Hautglanzes, insbesondere von fettiger Haut.
- die Verwendung von Tapioka enthaltenden kosmetischen Zubereitungen mit einem Lipidgehalt von 10 bis 35 Gewichts-%, bezogen auf das Gesamtgewicht der Zubereitung, zur Reduzierung des Hautglanzes, insbesondere von fettiger Haut.
- die Verwendung von Tapioka zum Erhalt der Hautfeuchte in kosmetischen Zubereitungen zur Reduzierung des Hautglanzes.
- die Verwendung von Tapioka in kosmetischen Zubereitungen mit einer Lipidphase, deren polare bis mittelpolare Bestandteile eine Grenzflächenspannung von 5-30 mN/m aufweisen, zur Reduzierung des Hautglanzes.

Zwar beschreibt die DE 101 57 542 Wasser-in-Silikonöl-Emulsionen enthaltend Tapioka, doch konnte diese Schrift nicht den Weg zur vorliegenden Erfindung weisen.

Tapioka (Echter Sago, Amylum Manihot. Manihotstärke, Mandiocastärke, Maniok, Caassavamehl oder Arrowroot, brasilianisch, Bahia-, Rio- oder Para-A.) ist die offizinelle brasilianische Stärke (Amilo brasil.). Es handelt sich um die aus den Wurzelknollen der in Südamerika u. Südostafrika heimischen Manihot-Arten, besonders esculenta Crantz (Jatropha manihot L., Manihot utilissima Pohl.), Euphorbiaceae, Maniok, Cassava u. Manihot dulcis Manihot (J. F. Gmel.) Pax var. multifida (Grah.) Pax, gewonnene Stärke (Fiedler, Lexikon der Hilfstoffe, Editio Cantor Verlag, Aulendorf, 5. Auflage, 2002).

Erfindungsgemäß vorteilhafte Verwendungen sind dadurch gekennzeichnet, dass Tapioka in der Zubereitung in einer Gesamtmenge von 0,1-25 Gewichts-%, bezogen auf das Gesamtgewicht der Zubereitung, enthalten ist.

Erfindungsgemäß bevorzugte Verwendungen sind dadurch gekennzeichnet, dass Tapioka in der Zubereitung in einer Gesamtmenge von 1-10 Gewichts-%, bezogen auf das Gesamtgewicht der Zubereitung, enthalten ist.

Zubereitungen gemäß der erfindungsgemäßen Verwendung können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen.

Erfindungsgemäß bevorzugt sind Verwendungen die dadurch gekennzeichnet sind, dass die Zubereitung in Form einer Emulsion vorliegt.

Dem entsprechend ist die erfindungsgemäße Verwendung in kosmetischen oder dermatologischen Zubereitungen wenn diese in Form einer Mehrfachemulsion, Mikroemulsion, Pickering-Emulsion, sprühbaren Emulsion oder eines Schaumes und ähnlichem vorliegen, erfindungsgemäß.

Erfindungsgemäße Emulsionen enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl, Macadamia-, Avocado- oder Jojobaöl, Dialkylether wie z.B. Di-n-octylether sowie Dialkylcarbonate wie beispielsweise Di-n-octylcarbonat.
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, 2-Methyl-1,3-propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wässrig-alkoholischen oder alkoholischen Gelen vorzugsweise ein Polyacrylat ist.

Die erfindungsgemäße Verwendung zeichnet sich erfindungsgemäß vorteilhaft auch dadurch aus, dass Tapioka in der Zubereitung die Klebrigkeit von Polyolen vermindert, wenn diese in einer Gesamtmenge von 0,1-25 Gewichts-%, bezogen auf das Gesamtgewicht der Zubereitung, in dieser vorhanden sind.

Erfindungsgemäß vorteilhafte Polyole sind dabei insbesondere Glycerin, Sorbitol, Harnstoff und Panthenol.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfume, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feucht haltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können erfindungsgemäß verwendete Wirkstoffkombinationen auch mit anderen Antioxidantien und/oder Radikalfängern kombiniert werden.

Vorteilhaft werden solche Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Phytin, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Ascorbinsäure und ihre Derivate wie Ascorbylpalmitat, Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die erfindungsgemäßen Zubereitungen können vorteilhaft Adstringentien enthalten, bevorzugt sind hier Chlorid-Salze und -Komplexe des Aluminiums und/oder Zirkoniums, besonders Aluminium Chlorohydrat.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- Ester der 2-Cyano-3,3-Diphenylacrylsäure, bevorzugt Ethylhexyl-2-cyano-3,3-diphenylacrylat,
- Diethylhexyl-butamidotriazon, 2,4,6-Trianilino-(p-carbo-2'-ethyl-1'-hexyloxy) -1,3,5-triazin .

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Gegenstand der Erfindung ist auch die Verwendung einer Kombination der erfindungsgemäß verwendeten Emulsionen mit mindestens einem UVB-Filter als Antioxidans bzw. die Verwendung einer Kombination der erfindungsgemäß verwendeten Wirkstoffkombinationen mit mindestens einem UVB-Filter als Antioxidans in einer kosmetischen oder dermatologischen Zubereitung.

Es kann auch von Vorteil sein, UVA-Filter einzusetzen, die üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)-propan-1,3-dion und um 1-Phenyl-3-(4'-isopropylphenyl)propan-1,3-dion.

Weiterhin vorteilhafte UVA-Filter entstammen der Gruppe der Triazine, so z.B. das 2,4-Bis-{[4-(2-Ethyl-hexyloxy)-2-hydroxy]-phenyl}-6-(4-methoxyphenyl)-1,3,5-triazin (Handelsbezeichnung Tinosorb® S) sowie der Gruppe der Triazole, wie z.B. das 2,2'-Methylen-bis- [6-2H-benzotriazol-2yl]-4-(1,1,3,3-tetramethylbutyl)phenol) (Handels-bezeichnung Tinosorb® M). Ein vorteilhafter wasserlöslicher UVA-Filter stellt das 2'-bis-(1,4-Phenylen)-1H-benzimidazol-4,6-disulfonsäure-Natriumsalz dar (Handelsbezeichnung Neo Heliopan AP®).

Es können die für die UVB-Kombination verwendeten Mengen eingesetzt werden.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).
Die Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |

Kosmetische Zubereitungen, die ein Hautreinigungsmittel darstellen, enthalten vorzugsweise mindestens eine anionische, nicht-ionische oder amphotere oberflächenaktive Substanz, oder auch Gemische aus solchen Substanzen, die erfindungsgemäß verwendeten Wirkstoffkombinationen im wässrigen Medium und Hilfsmittel, wie sie üblicherweise dafür verwendet werden.

Die oberflächenaktive Substanz bzw. die Gemische aus diesen Substanzen können in einer Konzentration zwischen 1 Gew.-% und 50 Gew.-% in dem Shampoonierungsmittel vorliegen.

Diese kosmetischen oder dermatologischen Zubereitungen können auch Aerosole mit den üblicherweise dafür verwendeten Hilfsmitteln darstellen.

Erfindungsgemäße wässrige kosmetische Reinigungsmittel oder für die wässrige Reinigung bestimmte wasserarme oder wasserfreie Reinigungsmittelkonzentrate können anionische, nichtionische und/oder amphotere Tenside enthalten, beispielsweise
- herkömmliche Seifen, z.B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobernsteinsäurehal bester
- Alkylethercarboxylate
- Eiweiß-Fettsäure-Kondensate
- Alkylbetaine und Amidobetaine
- Fettsäurealkanolamide
- Polyglycolether-Derivate

Kosmetische Zubereitungen, die kosmetische Reinigungszubereitungen für die Haut darstellen, können in flüssiger oder fester Form vorliegen. Sie enthalten neben erfindungsgemäß verwendeten Wirkstoffkombinationen vorzugsweise mindestens eine anionische, nicht-ionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Die erfindungsgemäßen Zusammensetzungen enthalten außer den vorgenannten Tensiden Wasser und gegebenenfalls die in der Kosmetik üblichen Zusatzstoffe, beispielsweise Parfum, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Die erfindungsgemäßen Verwendungen finden insbesondere in Haut- oder Gesichtscremes, Haut- oder Gesichtslotionen sowie Tages- oder Nachtcremes oder -lotionen Anwendung.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

| **W/O-Emulsionen mit Tapioka** | | | | | |
|---|---|---|---|---|---|
| | **1** | **2** | **3** | **4** | **5** |
| Triglycerindiisostearat | 1,0 | 0,5 | 0,25 | 2,0 | 3,0 |
| Diglycerindipolyhydrooystearat | 1,0 | 1,5 | 1,75 | 3,0 | 2,0 |
| Paraffinöl | 12,5 | 10,0 | 8,0 | 5,0 | 17,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | --- | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,2 | 0.05 | 0.4 | 0.3 | 2,0 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,04 | 0,15 | 0,05 | 0,03 | 0,4 |
| Benzylalkohol | 0,3 | 0,4 | 0,25 | 0,15 | --- |
| Tapioka | 1,5 | 0,3 | 0,5 | 1,0 | 5,0 |
| Wasser | ad 100 | ad 100 | ad 100 | ad 100 | ad 100 |

| **W/O-Emulsionen mit Tapioka** | | | | | |
|---|---|---|---|---|---|
| | **6** | **7** | **8** | **9** | **10** |
| PEG-30 Dipolyhydroxystearat | --- | 0,5 | 0,25 | --- | 3,0 |
| Lanolin Alcohol | 1,0 | 1,5 | 1,75 | 3,0 | -- |
| Paraffinöl | 12,5 | 10,0 | 8,0 | 5,0 | 17,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Natriumdehydracet | --- | --- | 0,05 | --- | --- |
| Kaliumsorbat | 0,3 | 0,4 | 0,25 | 0,15 | --- |
| Tapioka | 0,5 | 1,5 | 2,5 | 3,5 | 8,0 |
| Talkum | --- | --- | 0,05 | --- | 0,1 |
| Wasser | ad 100 | ad 100 | ad 100 | ad 100 | ad 100 |

| **W/S-Emulsion mit Tapioka** | | | | | |
|---|---|---|---|---|---|
| | **11** | **12** | **13** | **14** | **15** |
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 1,0 | 0,1 | 0,4 | 0,9 | 2,5 |
| Parfum | q,s, | q,s, | q,s, | q,s, | q,s, |
| Kaliumsorbat | 0,4 | 0,1 | 0,05 | 0,3 | 0,4 |
| Tapioka | 1,0 | 0,5 | 2,5 | 0,25 | 5,5 |
| Cetyldimethicon | 0,5 | --- | 0,7 | --- | --- |
| Benzylalkohol | --- | --- | 0,05 | --- | 0,1 |
| Wasser | ad 100 | ad 100 | ad 100 | ad 100 | ad 100 |

| **W/S-Emulsionen** | | | | | |
|---|---|---|---|---|---|
| | **16** | **17** | **18** | **19** | **20** |
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Milchsäure | 0,2 | 0,1 | 0,2 | --- | --- |
| Natriumlactat | 0,2 | 1,0 | 0.05 | --- | --- |
| Parfum | q,s, | q,s, | q,s, | q,s, | q,s, |
| Tapioka | 1,0 | 3,5 | 1,5 | 2,5 | 0,1 |
| Stearyldimethicon | 0,5 | --- | 0,7 | --- | --- |
| Dehydracetsäure | --- | --- | 0,05 | --- | 0,1 |
| modifizierte Stärke | --- | 2,5 | --- | 0,15 | -- |
| Wasser | ad 100 | ad 100 | ad 100 | ad 100 | ad 100 |

| **W/O-Emulsionen mit Tapioka** | | | | | |
|---|---|---|---|---|---|
| | **21** | **22** | **23** | **24** | **25** |
| PEG-22 Dodecyl Glycol Copolymer | 5,0 | 1,5 | 0,25 | --- | 3,0 |
| PEG-45 Dodecyl Glycol Polymer | 1,0 | 1,5 | 1,75 | 3,0 | -- |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Nachtkerzenöl | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Tapioka | 0,5 | 1,0 | 7,5 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Natriumcitrat | 0,2 | 0,1 | --- | --- | --- |
| Zitronensäure | 0,2 | 0,1 | --- | --- | --- |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,4 | 0,15 | 0,05 | 0,3 | 0,4 |
| Benzylalkohol | --- | --- | 0,05 | --- | 0,1 |
| Wasser | ad 100 | ad 100 | ad 100 | ad 100 | ad 100 |

| **W/O-Emulsionen mit Tapioka** | | | | | |
|---|---|---|---|---|---|
| | **26** | **27** | **28** | **29** | **30** |
| Polyglyceryl-2-dipolyhydroxystearat | 3,0 | --- | 0,25 | --- | 3,0 |
| Polyglyceryl-3 düsostearate | 1,0 | 3,5 | 1,75 | 2,5 | -- |
| PEG-40 sorbitanisostearate | --- | 2,5 | 0,5 | 3,5 | 3,0 |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Isopropylpalmitat | 0,5 | 1,0 | 0,75 | 3,0 | 0,25 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Tapioka | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,1 | 0,3 | 1,0 |
| Natriumcitrat | 0,2 | 0,3 | 0,2 | 1,5 | 0.8 |
| Parfum | q,s, | q,s, | q,s, | q,s, | q,s, |
| Capryl-/Caprinsäuretriglycerid | 2,0 | 2,5 | 3,0 | 5,0 | 0,5 |
| Kaliumsorbat | 0,24 | 0,15 | 0,05 | 0,3 | 0,4 |
| Wasser | ad 100 | ad 100 | ad 100 | ad 100 | ad 100 |

| **Silikon in Wasser-Emulsion** | | | | | |
|---|---|---|---|---|---|
| | **31** | **32** | **33** | **34** | **35** |
| Dimethiconcopolyol, Caprylic/Caprictriglycerid | 1,0 | 2,0 | 8,0 | 3,0 | 5,0 |
| Cyclomethicon | 12,5 | 15 | 25,0 | 10,0 | 7,5 |
| Dimethicon | 5,0 | 15,0 | 5,0 | 12,0 | 15,0 |
| Mineralöl | 0,5 | 0,75 | 1,0 | 2,0 | 0,25 |
| Phenyltrimethicon | 0,5 | 1,0 | 0,75 | 3,0 | 0,25 |
| Glycerin | 5,0 | 7,5 | 10,0 | 3,0 | 1,0 |
| Xanthan Gum | --- | 0,1 | -- | 0,25 | 1,0 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Tapioka | 0,5 | 2,5 | 1,0 | 0,75 | 3,5 |
| Wasser | ad 100 | ad 100 | ad 100 | ad 100 | ad 100 |

| **O/W-Emulsion mit Tapioka** | | | | | |
|---|---|---|---|---|---|
| | **36** | **37** | **38** | **39** | **40** |
| Glycerylsterat | 1,0 | --- | -- | 0,5 | 0,25 |
| Polyethylenglycol(40)stearat | 10,0 | --- | 5 | -- | -- |
| Triglycerinmethylglucosedistearat | --- | 5,5 | --- | --- | 2,5 |
| Sorbitanstearat | --- | 1,5 | 3 | --- | --- |
| Cyclomethicon | 2,5 | 15 | 8,0 | 5,0 | 7,5 |
| Dimethicon | 5,0 | 3,0 | 5,0 | 2,0 | 5,0 |
| Behenylalkohol | 1 | --- | 2 | 1 | --- |
| Stearylalkohol | --- | 1 | --- | 1 | --- |
| Tapioka | 5 | 10 | 15 | 20 | 2,5 |
| Cetylstearylalkohol | --- | --- | 1 | 1 | --- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Glycerin | 5 | 10 | 3 | 15 | 7,5 |
| modifizierte Stärke | --- | 2,5 | --- | 0,15 | -- |
| Wasser | ad 100 | ad 100 | ad 100 | ad 100 | ad 100 |

| **O/W-Emulsion mit Tapioka** | | | | | |
|---|---|---|---|---|---|
| | **41** | **42** | **43** | **44** | **45** |
| Polyethylenglycol(21)stearylether | 1 | -- | 2,5 | 2 | 1,5 |
| Polyethylenglycol(2)stearylether | 1 | -- | 5,5 | 3 | 7,5 |
| Cetearylglucosid | --- | 8 | --- | --- | --- |
| Behenylalkohol | 3 | 2 | --- | 1 | --- |
| Stearylalkohol | 3 | 2 | --- | 2 | --- |
| Cetylstearylalkohol | 3 | 4 | --- | --- | 2 |
| hydriertes Polyisobuten | 0,5 | 0,75 | 1,0 | 2,0 | 0,25 |
| Tapioka | 5 | 3,5 | 2,5 | 1,0 | 7,5 |
| Octyldodecanol | 0,5 | 1,0 | 0,75 | 3,0 | 0,25 |
| Glycerin | 5 | 10 | 15 | 3 | 7,5 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| modifizierte Stärke | 0,5 | --- | --- | 0,15 | -- |
| Wasser | ad 100 | ad 100 | ad 100 | ad 100 | ad 100 |

| **O/W-Emulsion mit Tapioka** | | | | | |
|---|---|---|---|---|---|
| | **46** | **47** | **48** | **49** | **50** |
| Glycerylsteratcitrat | 1,0 | 0,5 | 0,1 | 0,5 | 0,3 |
| Polyethylenglycol(20)cetearylether | 10,0 | 1,0 | 5 | -- | -- |
| Triglycerinmethylglucosedistearat | --- | --- | --- | --- | 2,5 |
| Ethylbutylacetylaminopropionat | 5 | --- | 15 | 20 | --- |
| Tapioka | 1,0 | 2,5 | 3,5 | 0,75 | 2,25 |
| Dimethicon | 0,5 | 3,0 | 0,75 | 1,5 | 0,2 |
| Behenylalkohol | 1 | --- | 2 | 1 | 0,2 |
| Dicaprylylcarbonat | 3 | 5 | 10 | 15 | 5 |
| Stearylalkohol | --- | --- | --- | 1 | 0,2 |
| Cetylstearylalkohol | --- | --- | 1 | 1 | 0,2 |
| Tocopherol | 0,5 | 0,5 | 0,75 | 0,25 | 0,1 |
| Octyldodecanol | 0,5 | --- | 0,75 | 3,0 | 0,25 |
| Panthenol | 0,5 | --- | 0,75 | 0,25 | 0,1 |
| Carbomer | 0,05 | 0,35 | 0,15 | 0,1 | --- |
| Parfum | q,s, | q,s, | q,s, | q,s, | q,s, |
| Caprylic/Capric Triglycerid | 1 | 5 | 3 | 5 | 10 |
| Methylparaben | 0,4 | 0,3 | 0,05 | 0,3 | 0,4 |
| Propylparaben | 0,3 | --- | 0,25 | 0,15 | --- |
| lodopropynylbutylcarbamat | --- | --- | 0,05 | --- | 0,1 |
| Phenoxyethanol | --- | 0,5 | --- | 0,15 | --- |
| Sorbitol | 10 | --- | -- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

| **O/W-Emulsion mit Tapioka** | | | | | |
|---|---|---|---|---|---|
| | **51** | **52** | **53** | **54** | **55** |
| Glycerylsterat | 2,6 | --- | 2,6 | 0,5 | 0,3 |
| Caprylic/Capric Triglycerid | --- | 0,6 | --- | --- | 1,5 |
| Cetylalkohol | --- | 2,5 | --- | --- | 2,0 |
| Glycerylstearatcitrat | --- | 2,0 | --- | --- | 3,0 |
| Cetystearylalkohol | 1,5 | --- | 2,5 | -- | -- |
| Stearylalkohol | --- | 2,5 | --- | 1,25 | --- |
| Polyethylenglycol(40)stearat | 0,8 | --- | 0,8 | --- | 2,5 |
| C12-15 Alkylbenzoat | 3,0 | --- | 3,0 | 2,0 | --- |
| Tapioka | 3,0 | 3,0 | 3,5 | 0,75 | 2,25 |
| Bienenwachs | 1,0 | 3,0 | 1,0 | 1,5 | 0,2 |
| Dicapryllyether | --- | 3,0 | --- | --- | --- |
| Octyltriazon | 1,5 | --- | 2 | 1 | 0,2 |
| BHT | 0,05 | 0,05 | 0,05 | 15 | 5 |
| Hydriertes Pflanzenöl | 1,5 | 3,0 | 1,5 | 1 | 0,2 |
| Hydriertes Kokosglycerid | --- | 1,5 | --- | --- | 0,5 |
| Tridecylstearat + Tridecyltrimellitat + Dipentaerythritylhexacaprylat/hexacaprat | --- | 1,2 | --- | --- | 2,5 |
| Benzophenon-3 | --- | --- | 3,5 | --- | --- |
| Ethylhexylzimtsäureester | --- | --- | 5,0 | --- | --- |
| Kokosglyceride | 3,0 | --- | 3,0 | 1 | 0,5 |
| Phenoxyethanol, Methyl-,Ethyl-, Butyl-,Isobutyl-,Propylparaben | 1,0 | 1,0 | 1,0 | 0,75 | 1,25 |
| EDTA, Na-Salz | --- | 1,0 | --- | --- | 1,0 |
| Glycerin | 10,0 | 11,6 | 10,0 | 3,0 | 12,9 |
| Dinatriumphenyldibenzimidazolsulfonat | 1,0 | --- | --- | 1,5 | 2,5 |
| Carbomer, Natrium-Salz | 0,1 | 0,2 | 0,1 | 0,1 | 0,3 |
| Parfum | q,s, | q,s, | q,s, | q,s, | q,s, |
| lodopropynylbutylcarbamat | 0,18 | --- | 0,18 | --- | --- |
| Cyclomethicon | 4,0 | 3,0 | 4,0 | --- | 3,5 |
| Tocopherolacetat | 0,5 | 0,5 | 0,5 | 0,15 | --- |
| Carrageenan | 0,1 | --- | 0,1 | --- | 0,1 |
| Phenylbenzimidazolsulfonsäure, Na-Salz | 1,0 | --- | 2,0 | 0,15 | --- |
| Kreatin | 1,0 | 1,0 | 1,0 | 1,5 | 3,0 |
| Kreatinin | 0,1 | 0,1 | 0,1 | 0,15 | 1,0 |
| Natriumascorbylphosphat | 0,05 | 0,05 | 0,05 | 5 | --- |
| Ethylhexylglycerin | --- | 0,5 | --- | --- | 0,35 |
| Panthenol | 1,4 | 1,4 | 1,4 | --- | --- |
| Retinylpalmitat | --- | 0,2 | --- | 0,5 | --- |
| Methylpropandiol | 2,0 | --- | 2,0 | --- | 5,0 |
| Ethylhexylcocoat | 2,0 | --- | 2,0 | --- | 4,0 |
| Coenzym Q10 | 0,01 | 0,01 | 0,01 | --- | 0,1 |
| Wasser | ad 100 | ad 100 | ad 100 | ad 100 | ad 100 |

## Patentansprüche

1. Verwendung von Tapioka in kosmetischen Zubereitungen zur Reduzierung des Hautglanzes.

2. Verwendung von Tapioka in kosmetischen Zubereitungen mit einem Lipidgehalt von 10-35 Gewichts-%, bezogen auf das Gesamtgewicht der Zubereitung, zur Reduzierung des Hautglanzes.

3. Verwendung von Tapioka in kosmetischen Zubereitungen mit einer Lipidphase, deren polare bis mittelpolare Bestandteile eine Grenzflächenspannung von 5-30 mN/m aufweisen, zur Reduzierung des Hautglanzes.

4. Verwendung von Tapioka enthaltenden kosmetischen Zubereitungen zur Reduzierung des Hautglanzes.

5. Verwendung von Tapioka zum Erhalt der Hautfeuchte in kosmetischen Zubereitungen zur Reduzierung des Hautglanzes.

6. Verwendung nach einem der vorhergehenden Ansprüche, **dadurch gekennzeichnet, dass** die Zubereitung in Form einer Emulsion vorliegt.

7. Verwendung nach einem der vorhergehenden Ansprüche, **dadurch gekennzeichnet, dass** Tapioka in der Zubereitung in einer Gesamtmenge von 0,1-25 Gewichts-%, bezogen auf das Gesamtgewicht der Zubereitung, enthalten ist.

8. Verwendung nach einem der vorhergehenden Ansprüche, **dadurch gekennzeichnet, dass** Tapioka in der Zubereitung in einer Gesamtmenge von 1-10 Gewichts-%, bezogen auf das Gesamtgewicht der Zubereitung, enthalten ist.

9. Verwendung nach einem der vorhergehenden Ansprüche, **dadurch gekennzeichnet, dass** Tapioka in der Zubereitung die Klebrigkeit von Polyolen in einer Gesamtmenge von 0,1-25 Gewichts-%, bezogen auf das Gesamtgewicht der Zubereitung, vermindert.

10. Verwendung nach Anspruch 9, **dadurch gekennzeichnet, dass** als Polyole Glycerin, Sorbitol, Harnstoff und/oder Panthenol eingesetzt werden.
